# EUROPEAN PATENT APPLICATION

(11) **EP 4 234 695 A1**
(43) Date of publication of application: **30.08.2023**
(21) Application number: 22158601.9
(22) Date of filing: 24.02.2022
(51) Int. Cl.: C12N 15/10

(54) **METHODS FOR THE SELECTIVE REMOVAL OF CONTAMINANTS DURING A PROCESS OF PURIFYING BIOLOGIC AGENTS**

(71) Applicant: Sartorius BIA Separations d.o.o., 5270 Ajdovscina (SI)
(72) Inventor: BOZIC, Klemen, Ajdovscina (SI); SEKIRNIK, Rok, Ajdovscina (SI); CERNIGOJ, Urh, Ajdovscina (SI)
(74) Representative: Müller-Boré & Partner Patentanwälte PartG mbB

(57) **Abstract**

The present invention relates to methods for the selective removal of contaminants during a process of purifying a biologic agent, comprising a step of subjecting a solution containing (i) said biologic agent and (ii) potential contaminants to a filtration step using a charge-neutral organic polymer filter in the presence of a kosmotropic agent, wherein said one or more contaminants are contaminants that bind to the charge-neutral organic polymer filter in the presence of said at least one kosmotropic agent, while the biologic agent does not bind to the charge-neutral organic polymer filter in the presence of said at least one kosmotropic agent. These methods combine a dual use of the filter for chemical and mechanical removal of contaminants in a single step. The present invention further relates to respective methods for the purification of a biologic agent, as well as respective uses of a charge-neutral organic polymer filter.

## Description

The present invention relates to methods for the selective removal of contaminants during a process of purifying a biologic agent, comprising a step of subjecting a solution containing (i) said biologic agent and (ii) potential contaminants to a filtration step using a charge-neutral organic polymer filter in the presence of a kosmotropic agent, wherein said one or more contaminants are contaminants that bind to the charge-neutral organic polymer filter in the presence of said at least one kosmotropic agent, while the biologic agent does not bind to the charge-neutral organic polymer filter in the presence of said at least one kosmotropic agent. These methods combine a dual use of the filter for chemical and mechanical removal of contaminants in a single step. The present invention further relates to respective methods for the purification of a biologic agent, as well as respective uses of a charge-neutral organic polymer filter.

Modern downstream bioprocessing addresses challenges in the purification of new classes of therapeutic modalities, such as monoclonal Antibodies (mAbs), plasmid DNA (pDNA), messenger RNA (mRNA), Virus Like Particles (VLP) or nanoplexes, as well as cell-based therapies (e.g. stem cell therapies). Since an important part of any current downstream processing scheme is based on chromatography, there is a constant need to improve the purification efficiency and to intensify the separation processes.

Examples in this respect are the production of plasmid DNA for use as a gene therapy vector, or as a raw material in production of viruses or mRNA, which consists of multiple filtration and chromatographic steps, as well as the production of recombinant proteins in e.g. *E. coli.*

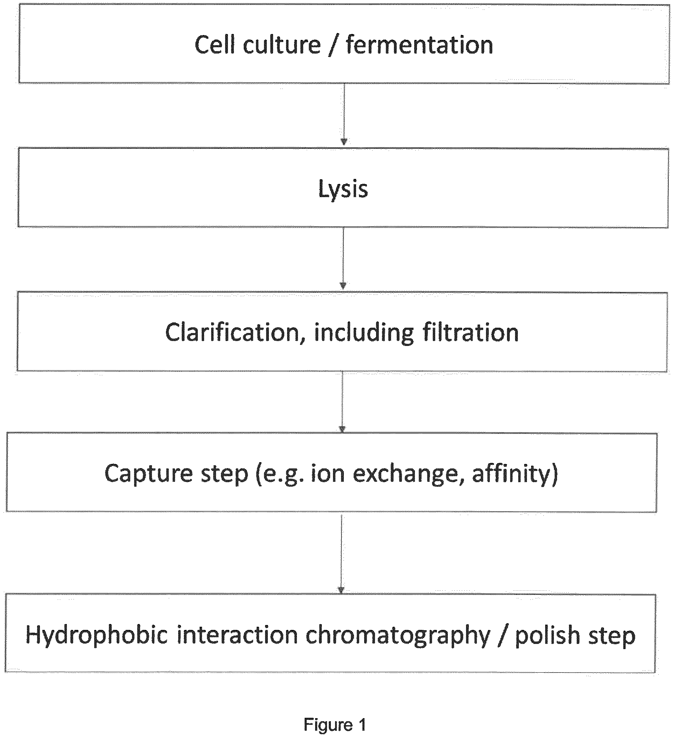

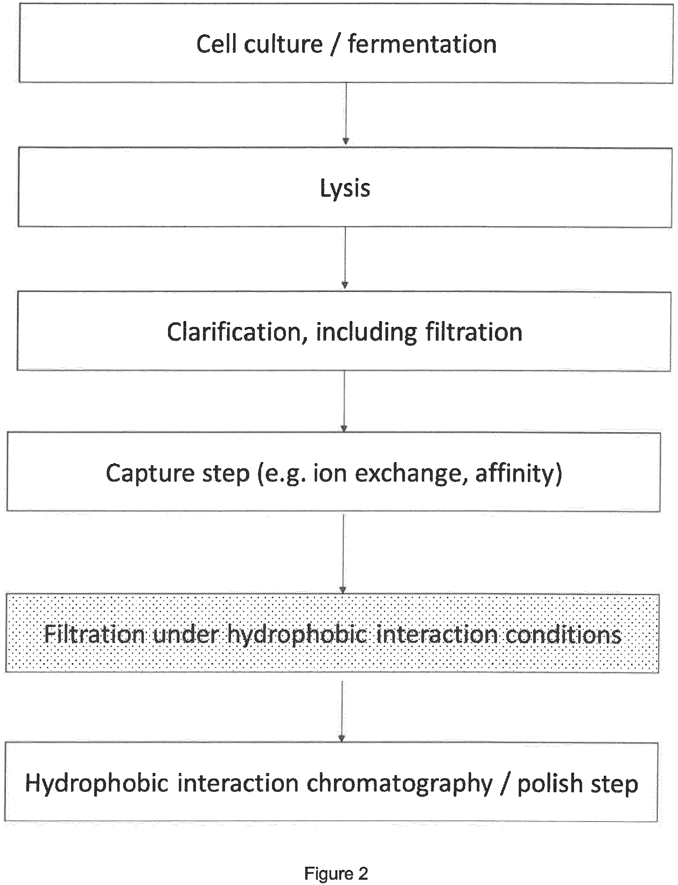

The isolation of biologic agents based on chromatography comprises multiple process steps, including but not limited to fermentation, lysis (for intracellular product; or extracellular product lysis can be avoided), clarification, capture chromatography and polishing chromatography (Fig. 1). An example of such a process is the purification of plasmid DNA, which consists of lysis of bacterial cells which releases the target molecule (e.g. pDNA), as well as a range of impurities, including host-cell proteins, host cell DNA, host cell RNA and endotoxins, which need to be removed by the purification process. Lysis is typically performed by addition of a strong-base and a surfactant, followed by neutralization and precipitation of RNA using calcium chloride, and filtration using a non-adsorbing filter to remove particulates/precipitate formed from cell lysate upon precipitation of RNA (impurity).

Plasmid is then captured from filtered neutralized bacterial lysate with a weak anion exchange chromatographic column, followed by polishing with hydrophobic interaction chromatography in the presence of kosmotropic salts (e.g. (NH₄)₂SO₄) to remove residual RNA, unwanted DNA isoforms, remains of genomic DNA, residual endotoxin and residual proteins (Fig. 1).

Production of plasmid DNA is required as a standalone gene therapy vector or a vaccine, or a raw material in production of viruses, mRNA, circular RNA or other therapeutic modalities produced by *in vitro* transcription from a DNA template. There is a high demand for the production of plasmids, with a consequent pressure on increasing the yield of the production/purification process.

Endotoxins are inherently hydrophobic; host-cell RNA and genomic (bacterial) DNA exhibit hydrophobic properties in the presence of kosmotropic agents, which are commonly used in downstream purification, in particular hydrophobic interaction chromatography. In addition to hydrophobic contaminants, the use of kosmotropic agents can lead to precipitation of biologic molecules, e.g. host cell proteins. Precipitates in turn lead to fouling of chromatographic columns.

Contaminants such as host-cell RNA, bacterial DNA and endotoxins would normally bind to the surface of hydrophobic interaction chromatography resin and compete for binding with the target molecule (e.g. pDNA); selective elution from HIC resin is achieved by modulation of the concentration of kosmotropic agent. Presence of such contaminants therefore decreases the binding capacity of HIC columns, and potentially decreases purity and/or yield of the target product.

Typically, pDNA elutes at higher concentration of salt than host cell RNA, though partial overlap in elution is common, leading to lower recovery of pDNA, which can partly co-purify with RNA.

Host-cell RNA and genomic DNA are typically removed by precipitation by CaCl₂, although the removal is partial. Residual host cell RNA, genomic DNA and endotoxins are removed by hydrophobic interaction chromatography and/or anion-exchange chromatography using chromatographic devices such as porous particles, membranes or monoliths. Despite multiple purification steps applied, contamination with residual host cell RNA, genomic DNA and endotoxins can lead to insufficient purity to meet regulatory specifications.

Proteins, RNA, bacterial DNA and endotoxin concentrations can be decreased with ammonium sulphate precipitation. This step further acts as a suitable conditioning step since the salt type and concentration (2-2.5 M) in the final pDNA-containing supernatant match the optimal requirements for a HIC feed. However, the precipitation alone is not sufficiently efficient to decrease the contaminants below the specification limits.

Accordingly, the technical problem underlying the present invention is the provision of means for improving the yield and/or purity in processes for the purification of biologic agents, in particular with respect to contaminating residual RNA, unwanted DNA isoforms, remains of genomic DNA, residual endotoxin and/or residual proteins.

The solution to the above technical problem is achieved by the embodiments characterized in the claims.

In particular, in a first aspect, the present invention relates to a method for the selective removal of one or more contaminants during a process of purifying a biologic agent, comprising a step of subjecting a solution containing said biologic agent and potential contaminants to a filtration step using a charge-neutral organic polymer filter in the presence of at least one kosmotropic agent, wherein said one or more contaminants are contaminants that bind to the charge-neutral organic polymer filter in the presence of said at least one kosmotropic agent, while the biologic agent does not bind to the charge-neutral organic polymer filter in the presence of said at least one kosmotropic agent.

As used herein, the term "selective removal" refers to the fact that removal of contaminants by way of the filtration step of the methods of the present invention is selective for said contaminants, *i.e*., said filtration step removes said contaminants but does not remove any other components that might be present in the above solution, or does not remove such other components to a substantial degree. In particular, the above filtration step does not remove the above biologic agent, or does not remove the biologic agent to a substantial degree. Preferably, at least 20% of contaminants, more preferably at least 50% of contaminants, more preferably at least 80% of contaminants are removed, while, preferably less than 5%, more preferably less than 2% of the above other components are removed.

The term "contaminants" as used herein is not particularly limited and includes any contaminants that might be relevant for a given process of purifying a biologic agent, provided said contaminants fulfil the above requirement of binding to the charge-neutral organic polymer filter in the presence of said at least one kosmotropic agent, while the biologic agent does not bind to the charge-neutral organic polymer filter in the presence of said at least one kosmotropic agent. In other words, said contaminants exhibit a higher hydrophobicity in the presence of said at least one kosmotropic agent compared to the hydrophobicity of the biologic agent in the presence of said at least one kosmotropic agent. In specific embodiments, the contaminants are contaminants derived from the host cells in which the biologic agent is produced, e.g. from bacterial cells. In further specific embodiments, the contaminants are selected from the group consisting of contaminating RNA species, host cell DNA (e.g. host cell genomic DNA, e.g. bacterial DNA), host cell proteins, endotoxins (*i.e*., bacterial lipopolysaccharides (LPS)), and mixtures thereof.

The term "during a process of purifying a biologic agent" indicates that the methods of the present invention can be performed as a part of a process of purifying a biologic agent. Respective processes are not particularly limited and include any purification processes in which contamination with contaminants may be relevant. By way of example, such processes include processes of purifying plasmid DNA (pDNA), processes of purifying recombinant proteins, and processes of purifying viral vectors, as known in the art.

The biologic agent to be purified in a process of which the methods of the present invention can be a part is not particularly limited and includes any biologic agents in the purification process of which contaminants may be relevant. As indicated above, in specific embodiments, the biologic agent is selected from the group consisting of recombinant proteins, viral vectors, viral vaccines, pDNA, DNA-based therapeutics, and RNA-based therapeutics (e.g. mRNA).

In specific embodiments, the biologic agent to be purified in a process of which the methods of the present invention can be a part has been produced in bacterial cells (such as, e.g., in *Escherichia coli).*

Charge-neutral organic polymer filters that can be used in the present invention are not particularly limited and include any charge-neutral organic polymer filters known in the art, provided said filters fulfil the requirement of binding the contaminants in the presence of said at least one kosmotropic agent, while not binding the biologic agents in the presence of the at least one kosmotropic agent. In other words, the charge-neutral organic polymer filter exhibits hydrophobicity in the presence of said at least one kosmotropic agent, specifically a hydrophobicity that allows for the binding of the contaminants to the filter, but does not allow for the binding of the biologic agent to the filter. Preferably, such charge-neutral organic polymer filters are selected from the group consisting of cellulose acetate filters, regenerated cellulose filters, polytetrafluoroethylene (PTFE) filters, polypropylene filters, polyethylene filters, polyamide filters, and unmodified polyethersulfone (PES) filters, wherein PES filters are particularly preferred. The pore size of respective filters is not particularly limited and includes pore sizes in the range of 0.2 µm to 50 µm, e.g., 0.45 µm. By way of example, such membranes include Sartorius Minisart 0.45 µm PES filters.

The present invention uses charge-neutral organic polymer filters in the presence of at least one kosmotropic agent, *i.e.,* under specific hydrophobic interaction conditions (HIC), wherein these conditions enable the selective binding of the contaminants to the filter. Such HIC include the presence of a kosmotropic agent in a concentration of at least 0.5 M, preferably at least 1 M, more preferably of 1 to 3 M, more preferably 2 to 3 M, e.g. about 2.5 M. Further, such HIC can include the additional presence of NaCI in a concentration of at least 0.5 M, preferably at least 1 M, more preferably of 1 to 2 M, e.g. about 1.5 M. Moreover, such HIC can further include a conductivity of at least 50 mSi/cm and/or a pH in the range of pH 6 to pH 9, preferably pH 7 to pH 8. Suitable kosmotropic agents are not particularly limited and are known in the art. Preferably, the kosmotropic agent is a kosmotropic salt of ammonium, potassium, cesium, sodium, fluorides, chlorides, sulfates, carbonates, phosphates including pyrophosphates, carboxylates, and combinations thereof. In a specific preferred embodiment, the kosmotropic agent is ammonium sulfate ((NH₄)₂SO₄). In another specific preferred embodiment, the kosmotropic agent is sodium chloride (NaCI).

According to preferred embodiments, the filtration step of the methods of the present invention is performed prior to any HIC chromatography steps that might be part of the process of purifying the biologic agent. In this context, many conventional methods for the purification of biologic agents contain a step of hydrophobic interaction chromatography in the presence of kosmotropic salts using chromatography columns. Of note, these HIC chromatography steps are different from the filtration step of the methods of the present invention, in that the former use specific column materials without filter functionality and not any charge-neutral organic polymer filters. Suitable HIC chromatography materials are not particularly limited and are known in the art. Preferably, the HIC chromatography material includes solid phases with surfaces possessing a butyl (C4), phenyl, octyl (C8), pyridine, or ether ligand.

In preferred embodiments, the filtration step of the methods of the present invention performs a dual function of mechanical filter and chemical adsorber of contaminants with a hydrophobicity greater than the target biologic agent at a given concentration of the at least one kosmotropic agent. Thus, the charge-neutral organic polymer filter simultaneously performs mechanical removal of particulates while acting as the chemical adsorber. This filtration step can be, but is not limited to being performed in-line with such HIC chromatography steps, *i.e.,* the filter outlet is connected to the inlet of a respective chromatography column. Preferably, said filtration step is performed immediately upstream of said HIC chromatography steps, *i.e.,* the outlet of the filter is directly connected to the inlet of a respective chromatography column.

In a second aspect, the present invention relates to a method for the purification of a biologic agent, comprising the step of performing the method according to the first aspect of the present invention, thereby increasing the yield and/or purity of said biologic agent.

In this aspect, all relevant definitions and limitations given for the methods according to the first aspect of the present invention equally apply. In particular, the contaminants, purification processes, biologic agents, charge-neutral organic polymer filters, hydrophobic interaction conditions (HIC), and kosmotropic agents are as defined above for the first aspect of the present invention.

In a third aspect, the present invention relates to the use of a charge-neutral organic polymer filter for the selective removal of contaminants during a process of purifying a biologic agent, wherein said charge-neutral organic polymer filter is used in the presence of at least one kosmotropic agent.

In this aspect, all relevant definitions and limitations given for the methods according to the first aspect of the present invention equally apply. In particular, the contaminants, purification processes, biologic agents, charge-neutral organic polymer filters, hydrophobic interaction conditions (HIC), and kosmotropic agents are as defined above for the first aspect of the present invention.

As used herein, the term "comprising"/"comprises" expressly includes the terms "consisting essentially of'/"consists essentially of" and "consisting of"/"consists of", *i.e.,* all of said terms are interchangeable with each other herein.

Further, as used herein, the term "about" preferably represents a modifier of the specified value of ± 10%, more preferably ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention provides means for the selective removal of contaminants during a process of purifying a biologic agent, wherein kosmotropic agents used within a defined concentration window lead the host cell contaminants, e.g. RNA, endotoxins and residual genomic DNA to expel water from the surface and bind to the relatively hydrophobic surface of a charge-neutral organic polymer filter, while the biologic agent, e.g. pDNA monomers, do not exhibit sufficiently hydrophobic behavior to bind to the filter, consequently passing through the filter. Contaminants are thus removed from the HIC load by the filter; filtrate is applied directly onto a hydrophobic interaction resin, under hydrophobic interaction mode. This leads to binding of the biologic agent onto the binding sites of the chromatographic column; if contaminants were present in the load, these would also bind to the column, thereby competing with the biologic agent for binding sites and decreasing the binding capacity for the biologic agent. Filtration of HIC load according to the present invention is thus an important contributor to high recovery of target product, since it means that the contaminants do not bind to the surface of the chromatography resin and frees the binding capacity for binding of the biologic agent.

Applying this unusual feature of charge-neutral organic polymer filters has multiple benefits for the economics and efficiency of the downstream purification of biologics. In particular, it provides a cost-effective purification approach for removal of a critical contaminant that would otherwise be removed by chromatography, e.g. residual RNA, genomic DNA, and/or endotoxins. Further, due to prior removal of this contaminant, recovery of HIC chromatography is increased by extending the acceptable elution window of the biologic agent. This leads to broader acceptance conditions and potentially lower number of failed batches during production. Furthermore, due to prior removal of this contaminant, binding capacity of HIC chromatographic resin is increased by increasing the binding surface available for the biologic agent which would otherwise be occupied by chemically related species. This decreases the size of the column required for HIC chromatography step, *i.e.,* decreases the cost of the chromatography step. Moreover, particulates formed in the presence of kosmotropic agents are concurrently mechanically removed by filtration, avoiding HIC column fouling.

Thus, the present invention advantageously increases the performance of the downstream chromatography step as follows: contaminants are removed by filtration in the presence of a kosmotropic agent, thereby increasing the recovery of HIC chromatography step, and consequently increasing the total process recovery. Additionally, aggregation of feed-stream upstream of the HIC chromatography step is removed by the filter functioning in its conventional function as a mechanical filter.

In this context, charge-neutral organic polymer filters are conventionally used in purification processes for biologic agents for the removal of particulates (*i.e.,* acting as a mechanical barrier for particles larger than the filter pores) in a non-adsorbing fashion, wherein adsorption onto a filter device was typically considered a disadvantage, due to potential removal of target molecule and consequent lowering of process yield.

Thus, the present invention represents a counterintuitive use of charge-neutral organic polymer filters, such as e.g. PES filters. In particular, the adsorption onto such filters was considered a disadvantage by persons skilled in the art, due to potential binding of target product. By way of the present invention, this disadvantage is turned into an advantage by combining several features that have not been previously applied in the purification of biologic agents. Specifically, the present invention employs a commonly used filter used for the removal of particulates, with an added feature that the hydrophobic nature of the surface is augmented by matrices comprised of kosmotropic agents at high concentrations.

A further unusual feature of the invention is the use of the filter immediately upstream of a commonly used hydrophobic interaction chromatography step. This is counterintuitive because filters are not typically used between two chromatography steps. In this case, application of a filtration step in the presence of kosmotropic agents is desirable for purpose of chemical, as well as concurrent mechanical, removal of contaminants.

Another unusual feature of the present invention is that it requires no additional buffer exchange steps that would not be used in a standard purification process. The method requires buffer compositions identical to downstream hydrophobic interaction chromatography step. This is counterintuitive because PES filters are not expected to adsorb nucleic acid species under the experimental conditions used.

The figures show:
Figure 1:
   Standard purification process scheme for purification of biologics, e.g. plasmid DNA.
Figure 2:
   Modification of a standard purification process scheme for purification of biologics, e.g. plasmid DNA, by including the present invention, *i.e.*, filtration under hydrophobic interaction conditions in the presence of kosmotropic agents.
Figure 3:
   Examples of HIC chromatography (polishing) step in the absence and presence of upstream PES filtration.
   A: C4 HLD (HIC) chromatography on plasmid DNA in absence of PES filtration.
   B: C4 HLD (HIC) chromatography on plasmid DNA after PES filtration in presence of kosmotropic salt (NH₄)₂SO₄. Arrows indicate differences in elution profiles in absence/presence of filtration. FT: flow-through, E1-E3: elution fractions 1-3.
Figure 4:
   Agarose gel electrophoresis (AGE) of plasmid pAAV2/8 (7.3 kbp) fractions after Case 1 and Case 2 C4 HLD (HIC) chromatographic steps. FT: flow-through, E1-E3: elution fractions E1-E3, 1: SC ladder (NEB), L-: load before PES filtration, L+: load after PES filtration, W: PES filter wash with ddH₂O, 2: Riboruler (ThermoFischer Scientific).
Figure 5:
   HPLC chromatograms (CIMac pDNA) of plasmid pAAV2/8 (7.3 kbp) fractions after Case 1 (A) and Case 2 (B) C4 HLD chromatographic steps. FT: flow-through, E1-E3: elution fractions E1-E3.
Figure 6:
   HPLC chromatograms (CIMac pDNA) of plasmid pAAV2/9n (7.3 kbp) fractions from C4 HLD chromatographic step - sample preparation and filtration. PES filter wash: filtrate collected after PES filter was washed with ddH₂O. Right: AGE of fractions 1: load before PES, 2: load after PES, 3: PES filter wash.
Figure 7:
   HPLC chromatograms (CIMac pDNA) of plasmid pAAV2/5 (7.4 kbp) fractions from C4 HLD chromatographic step - sample preparation and filtration. PES filter wash: filtrate collected after PES filter was washed with ddH₂O. Right: AGE of fractions 1: load before PES, 2: PES filter wash, 3: load after PES.
Figure 8:
   HPLC chromatograms (CIMac pDNA) of plasmid pUCBS4.7 (4.7 kbp) fractions from C4 HLD chromatographic step - sample preparation and filtration. PES filter wash: filtrate collected after PES filter was washed with ddH₂O. Right: AGE of fractions 1: load before PES, 2: load after PES, 3: PES filter wash.
Figure 9:
   *Limulus* amoebocyte lysate (LAL) test results for removal of endotoxins by PES filtration in presence of kosmotropic agent. Plasmid sample was formulated in hydrophobic interaction (HIC) conditions (>1 M AS or >1 M NaCI). When filtered through PES filter, LAL shows significant (>30x) removal of endotoxins. Endotoxins are released from PES filter upon filter wash. Samples formulated under normal aqueous conditions show at best 2x endotoxin clearance (data not shown).

The present invention will be further illustrated by the following example without being limited thereto.

### Example

### Material and methods:

Plasmid DNA was prepared from *E. coli cell* paste up to DEAE (diethylaminoethyl) eluate stage as known in the art. DEAE eluate containing plasmid DNA and contaminants (in 50 mM TRIS, 10 mM EDTA, ca. 0.75 M NaCl) was prepared for HIC chromatography in the presence and absence of PES filtration as follows:
- Addition of MPC (see Table 1 below) to increase ammonium sulfate (AS) conc. to 2 M
- Additional dilution with MPL (2 M AS) to further reduce the effect of NaCl from DEAE eluate.

### Case 1: C4 chromatography in the absence of PES filtration

▪ Load the prepared sample in 2 M AS through system pump
▪ Wash with MPL (2 M AS) to return UV signal back to the baseline (ca. 8 mL)
▪ 3 min linear gradient from MPL (2 M AS) to MPA (without AS)
▪ 10 CV ddH₂O
▪ 10 CV 1 M NaOH, contact time in 1 M NaOH: 15 min (cf. column preparation in Table 1, below)

### Case 2: C4 chromatography in the presence of PES filtration

Sample was prepared as for Case 1, with an additional filtration step prior to C4 load as follows:
Before starting a run, the sample formulated in 2 M AS (see sample preparation above) was filtered through Minisart^{®} PES Syringe Filter, 0.45 µm, Sartorius. Filter was then rinsed with MPL to improve pDNA recovery from the filter. After filtration, the filter was washed with ddH₂O and filtrate collected as PES filter wash.
For HPLC analytics, samples were diluted 4x (with exception of E3 and PES filter wash, which were diluted 2x) with MPA for CIMac pDNA analytics before injection to reduce AS conc. enough to ensure binding conditions for pDNA.

**Table 1: HPLC system, column, filter and mobile phases**

| | |
|---|---|
| HPLC System | Akta Purifier 10 |
| Column | CIM C4 HLD bed volume 0.1 mL |
| Flow | 2 mL/min |
| UV | 260 nm, 280 nm, 10 mm optical path length |
| Mobile phase A (MPA) | 50 mM Tris, 10 mM EDTA, pH 7.21, cond. 5,145 mS/cm |
| Mobile phase C (MPC) | 50 mM Tris, 10 mM EDTA, 3,7 M (NH₄)₂SO₄(AS), pH 7.20, cond. |
| | 271,74 mS/cm |
| MPL (loading mobile phase) | 50 mM Tris, 10 mM EDTA, 2 M (NH₄)₂SO₄(AS), pH 7.18, cond. |
| | 222 mS/cm |
| Column preparation before each run | 10 CV 1 M NaOH (CIP), contact time in 1 M NaOH: 15 min |
| | 10 CV ddH₂O |
| | 20 CV MPA, contact time in MPA: 10 min |
| | 20 CV MPL → continue with load |
| PES filter | Minisart^{®} PES Syringe Filter, 0.45 µm, Sartorius |

### Results and discussion:

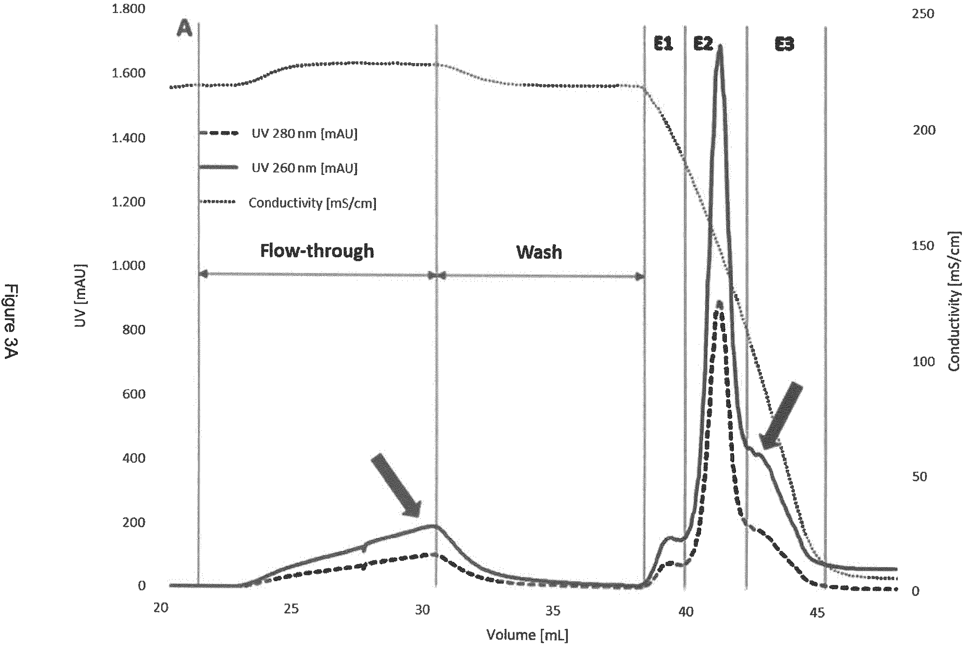

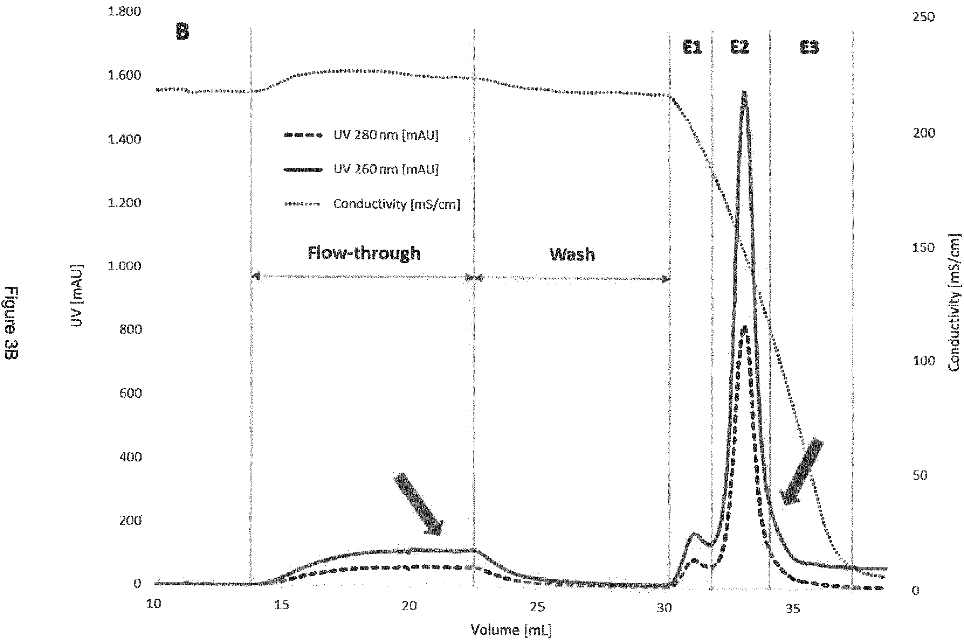

Comparison of chromatograms of C4 chromatographic purification of Case 1 (Fig. 3A) and Case 2 (Fig. 3B) reveals a difference in UV signal for flow-through (FT) between unfiltered and PES-filtered C4 loads, indicating higher impurity load on C4 in absence of filtration.

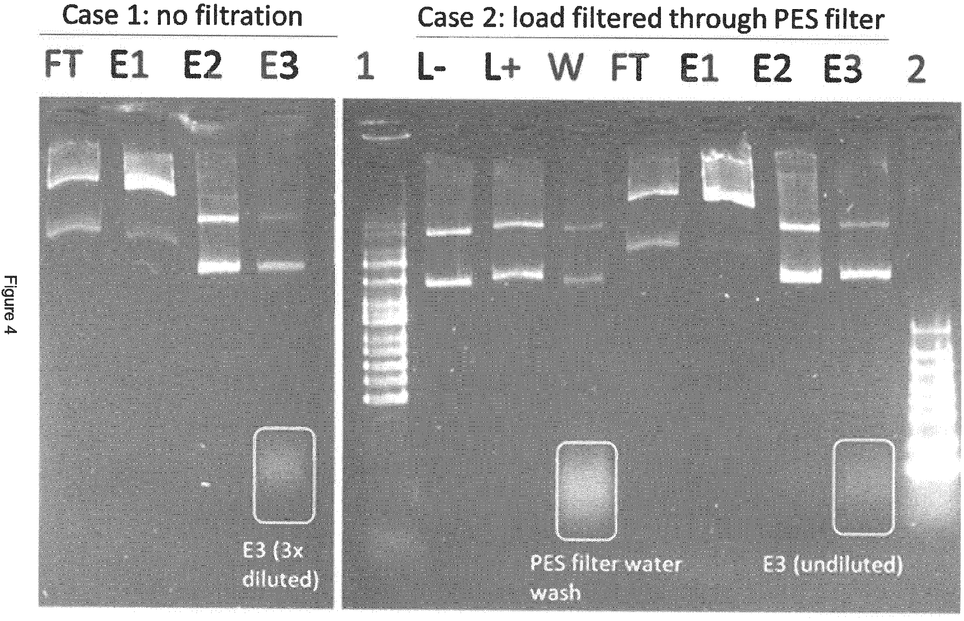

The difference in elution profile of elution peaks can be explained as follows: in the absence of PES filtration, additional impurity elutes from C4, seen as an elution shoulder (E3 in Fig. 3A). The peak is identified as RNA by agarose gel electrophoresis (cf. Fig. 4 for Case 1). As a consequence of co-elution of impurities, the elution fraction of plasmid (E2) needs to be collected separately from E3 to minimize contamination with RNA present in E3.

PES filtration prior to C4 chromatography leads to an absence of a peak eluting as E3 (Fig. 3B). This allows the elution window of pDNA to be extended, thereby increasing recovery of pure plasmid.

Agarose gel electrophoresis (AGE) of fractions collected for runs Case 1 and Case 2 (Fig. 4) shows significant presence of RNA impurities eluting from PES filter upon filter wash with ddH₂O (fraction W, Fig. 4). Furthermore, the RNA signal is strong in E3 collected for Case 1, whereas an RNA peak is not detectable in elution fractions (E1-E3) for Case 2.

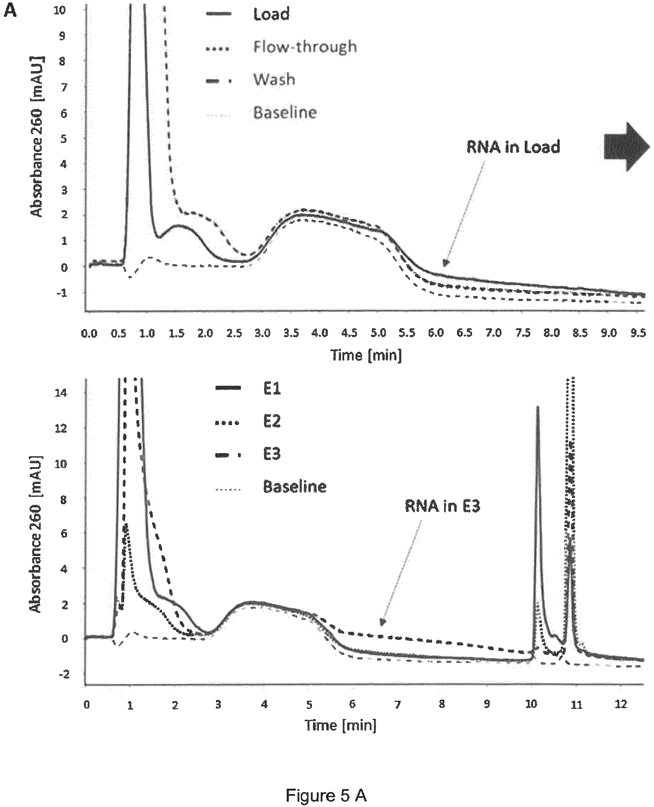

In HPLC analytics of Case 1 fractions (Fig. 5A), RNA is detected on CIMac pDNA column as a continuous, rather than discrete, increased in signal between retention times 4-10 min (heterogeneous population of short RNAs). Increased RNA signal is seen only in LOAD, not in FT and WASH, indicating that RNA binds to C4 HLD. RNA elutes from C4 in E3. A very wide RNA peak co-elutes with pDNA peaks (oc, sc isoforms).

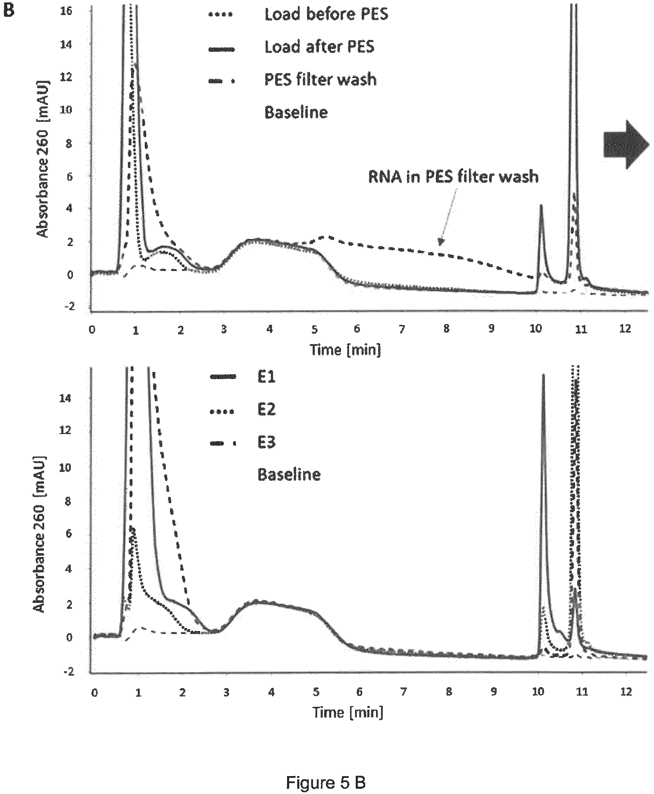

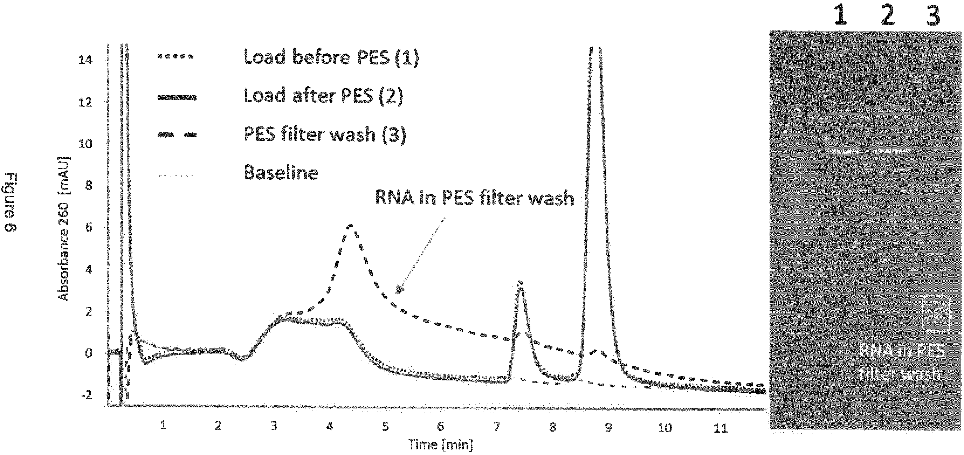

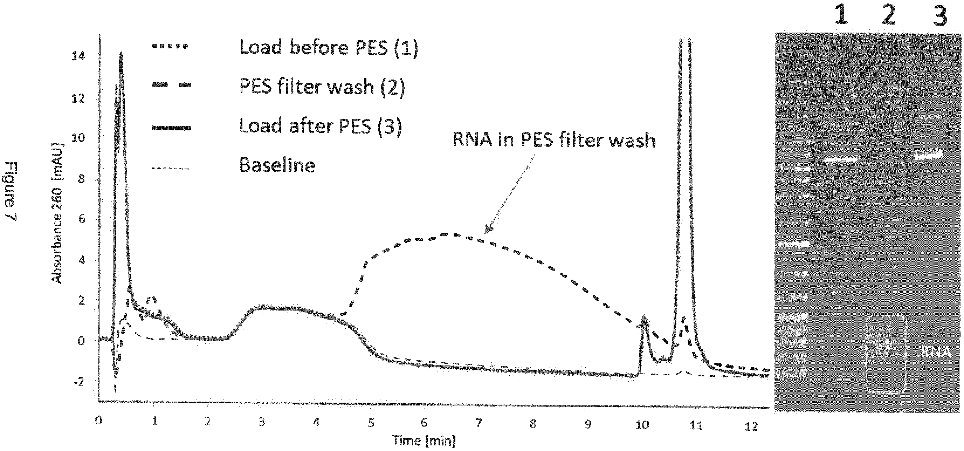

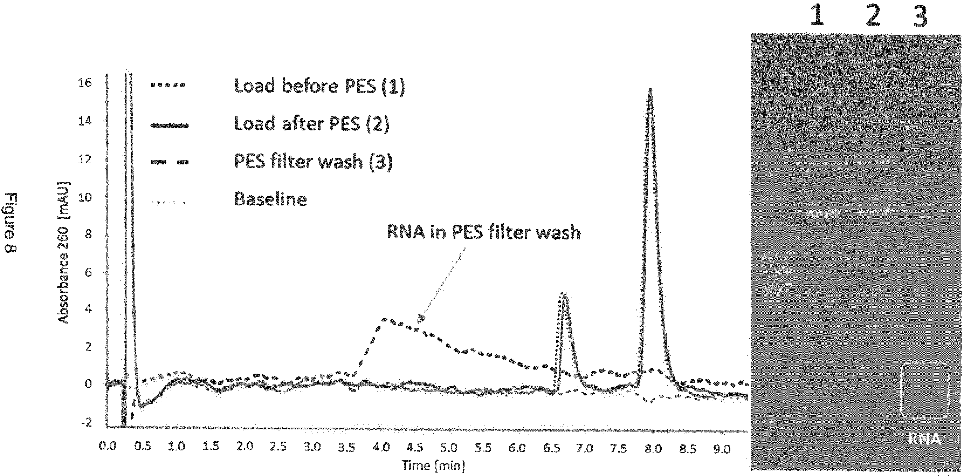

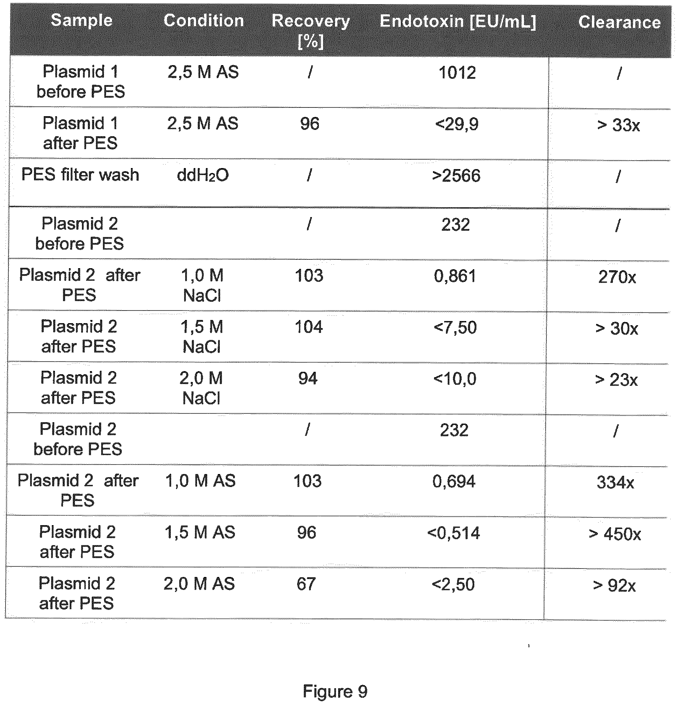

In Case 2 (Fig. 5B), the difference in RNA area between load before and after PES is not clearly recognizable by HPLC. RNA binds to PES filter in 2.0 M AS, as demonstrated by a high increase in RNA signal in filter wash with ddH₂O. Very wide RNA peak co-elutes with pDNA peaks (oc, sc isoforms). No RNA seen in E3, predominant elution of sc plasmid isoform.

## Claims

1. A method for the selective removal of one or more contaminants during a process of purifying a biologic agent, comprising
a step of subjecting a solution containing said biologic agent and potential contaminants to a filtration step using a charge-neutral organic polymer filter in the presence of at least one kosmotropic agent,
wherein said one or more contaminants are contaminants that bind to the charge-neutral organic polymer filter in the presence of said at least one kosmotropic agent, while the biologic agent does not bind to the charge-neutral organic polymer filter in the presence of said at least one kosmotropic agent.

2. The method of claim 1, wherein the one or more contaminants are host cell-derived contaminants.

3. The method of claim 1 or claim 2, wherein the one or more contaminants are selected from the group consisting of RNA, DNA, proteins, endotoxins, and mixtures thereof.

4. The method of any one of claims 1 to 3, wherein the biologic agent is selected from the group consisting of recombinant proteins, viral vectors, viral vaccines, plasmid DNA (pDNA), DNA-based therapeutics, and RNA-based therapeutics.

5. The method of any one of claims 1 to 4, wherein the charge-neutral organic polymer filter is selected from the group consisting of cellulose acetate filters, regenerated cellulose filters, polytetrafluoroethylene (PTFE) filters, polypropylene filters, polyethylene filters, polyamide filters, and unmodified polyethersulfone (PES) filters.

6. The method of any one of claims 1 to 5, wherein the at least one kosmotropic agent is present in said solution in a concentration of at least 0.5 M.

7. The method of claim 6, wherein said solution exhibits a conductivity of at least 50 mSi/cm and/or a pH in the range of pH 6 to pH 9.

8. The method of any one of claims 1 to 7, wherein the kosmotropic agent is selected from the group consisting of kosmotropic salts of ammonium, potassium, cesium, sodium, fluorides, chlorides, sulfates, carbonates, phosphates including pyrophosphates, carboxylates, and combinations thereof.

9. The method of claim 8, wherein the kosmotropic salt is ammonium sulfate ((NH₄)₂SO₄) or sodium chloride (NaCI).

10. The method of any one of claims 1 to 9, wherein the charge-neutral organic polymer filter simultaneously performs mechanical removal of particulates.

11. The method of any one of claims 1 to 10, wherein said filtration step is performed prior to any HIC chromatography steps that might be part of said process of purifying the biologic agent.

12. The method of claim 11, wherein said filtration step is performed in-line with said HIC chromatography steps, preferably wherein said filtration step is performed immediately upstream of said HIC chromatography steps.

13. A method for the purification of a biologic agent, comprising the step of performing the method as defined in any one of claims 1 to 12, thereby increasing the yield and/or purity of said biologic agent.

14. Use of a charge-neutral organic polymer filter for the selective removal of contaminants during a process of purifying a biologic agent, wherein said filter is used in the presence of at least one kosmotropic agent.
